# FASCICULE DE BREVET EUROPEEN

(11) **EP 1 100 528 B1**
(45) Date de publication et mention de la délivrance du brevet: **16.10.2002**
(21) Numéro de dépôt: 99936659.4
(22) Date de dépôt: 30.07.1999
(51) Int. Cl.: A61K 38/21, A61K 33/36, A61K 38/06, A61K 31/285, A61P 35/00

(54) **UTILISATION DE NOUVEAUX AGENTS INDUCTEURS DE MORT CELLULAIRE EN SYNERGIE AVEC LES INTERFERONS**
VERWENDUNG VON NEUEN ZELLTOD INDUCIERENDEN MITTELN IM SYNERGISMUS MIT INTERFERON
USE OF NOVEL AGENTS INDUCING CELL DEATH IN SYNERGY WITH INTERFERONS

(30) Priorité: 31.07.1998 FR 9809886
(43) Date de publication de la demande: 23.05.2001
(73) Titulaire: INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75016 Paris (FR)
(72) Inventeur: KOKEN, Marcel, 15, allée Darius Milhaud, 75019 Paris (FR); QUIGNON, Frédérique, F-75011 Paris (FR); DE THE, Hugues, F-75007 Paris (FR); AMEISEN, Jean-Claude, F-75005 Paris (FR); DE BELS, Frédéric, 75020 Paris (FR)
(74) Mandataire: Retzler, Charlotte
(86) Numéro de dépôt international: FR9901901
(87) Numéro de publication internationale: WO00007616

(56) Documents cités:
- WO-A-99/55344
- BYRD J.C. ET AL: "Old and new therapies in chronic lymphocytic leukemia: Now is the time for a reassessment of therapeutic goals" SEMINARS IN ONCOLOGY(SEMIN. ONCOL.), 25/1 (65-74), XP002104744 United States
- SOIGNET S ET AL: "Clinical and laboratory studies in leukemia of an organic arsenical, melarsoprol (Meeting abstract)." PROC ANNU MEET AM SOC CLIN ONCOL;16:A10 1997, XP002104745
- KONIG ANDREA_(A) ROBERTA: "Comparative activity of melarsoprol and arsenic trioxide in chronic B-cell leukemia lines." BLOOD, 1997, XP002095650
- CHEMICAL ABSTRACTS, vol. 124, no. 23, 3 juin 1996 (1996-06-03) Columbus, Ohio, US; abstract no. 307527, SLEE, ELIZABETH A. ET AL: "Benzyloxycarbonyl -Val-Ala-Asp (OMe) fluoromethylketone (Z-VAD.FMK) inhibits apoptosis by blocking the processing of CPP32--" XP002104746 & BIOCHEM. J. (1996), 315(1), 21-4 CODEN: BIJOAK;ISSN: 0264-6021,1996,

## Description

La présente invention concerne l'utilisation de nouveaux agents inducteurs de mort cellulaire, et en particulier d'un agent permettant la surexpresssion de la protéine PML sur les corps nucléaires, en association avec des interférons, pour induire la mort de cellules indésirables.

Les corps nucléaires sont des structures associées à la matrice nucléaire, de fonction inconnue, et qui contiennent un certain nombre de protéines incluant PML, Sp100, ISG20, PIC-1/SUMO-1, lysp100, PLZF, Int-6, CBP, Rb, RFP et la protéine P ribosomale (Lamond et al, 1998). Le gène codant pour la protéine PML (pour "ProMyelocytic Leukemia") a été identifié à partir de sa fusion avec le gène *RAR*α (récepteur nucléaire de l'acide rétinoïque) dans la translocation t(15;17) trouvée chez des patients atteints de leucémie promyélocytique aiguë ("Acute Promyelocytic Leukemia" - APL). Ce gène *PML* est un gène cible des interférons, et sa surexpression provoque un arrêt de la croissance de certaines lignées cellulaires (Koken et al, 1995). Dans les cellules malignes APL, la protéine PML n'est pas localisée sur les corps nucléaires mais délocalisée du fait de l'expression de PML-RARα. L'oxyde d'arsenic induit le retour de PML vers sa localisation normale ainsi que la mort de la cellule. Dans les cellules normales non APL, où la localisation de PML est normale, l'arsenic induit l'agrégation de PML vers de larges corps modifiés, mais le phénomène ne s'accompagne pas de mort cellulaire (Zhu et al, 1997).

Les auteurs de l'invention ont à présent découvert que la surexpression de la protéine PML localisée sur les corps nucléaires provoque la mort de la cellule, par un mécanisme original différent de celui de l'apoptose induite par les caspases.

La conséquence majeure de cette découverte est qu'une substance favorisant l'adressage de la protéine PML vers les corps nucléaires et/ou sa stabilisation, est particulièrement utile pour induire la mort de cellules indésirables.

Lesdites substances qui induisent l'adressage de la protéine PML vers les corps nucléaires et/ou sa stabilisation, peuvent être identifiées par des tests standard connus de l'homme du métier, la mesure du transit intracellulaire entre les fractions cytoplasmique et nucléoplasmique et la fraction associée aux corps nucléaires et la stabilisation de la protéine PML pouvant être notamment réalisée par Western Blot.

Lesdites cellules indésirables peuvent être notamment des cellules d'une tumeur, des cellules infectées par un virus, un parasite ou une bactérie, des cellules immunitaires participant à une réaction immunitaire inappropriée, des cellules génétiquement altérées, des cellules sénescentes ou hyperplasiques

Par "tumeur", on entend toute prolifération cellulaire indésirable, bénigne ou maligne, incluant notamment les cancers solides et les leucémies et lymphomes. Parmi les tumeurs malignes, on peut citer en particulier les leucémies myéloïdes chroniques et les leucémies lymphoïdes T de l'adulte (ATL) et les mélanomes.

La présente invention a donc pour objet l'utilisation d'au moins une substance favorisant l'adressage de la protéine PML vers les corps nucléaires et/ou sa stabilisation pour la fabrication d'un médicament destiné à induire la mort de cellules indésirables.

L'expression de la protéine PML étant induite par des interférons, la présence d'interférons, qu'ils soient d'origine endogène ou administrés au patient de manière simultanée ou séquentielle, est nécessaire à l'efficacité du traitement envisagé.

De manière surprenante, les auteurs de la présente invention ont plus particulièrement découvert que le zVAD (benzyloxycarbonyl-Val-Ala-Asp(O-méthyl)-fluorométhylcétone) d'une part stabilise la protéine PML et d'autre part accélère la mort cellulaire induite par les interférons.

Or, le zVAD est initialement connu comme inhibiteur des caspases, protéases impliquées dans le processus d'apoptose (Salvesen et al, 1997). Des études (Mc Carthy et al, 1997) ont en outre montré que le zVAD empêchait ou retardait fortement la mort cellulaire. La découverte des auteurs de la présente invention, selon laquelle le zVAD ne bloque pas la mort cellulaire induite par les interférons mais au contraire l'accélère, va donc à l'encontre des résultats que pouvait attendre l'homme du métier.

La présente invention a plus particulièrement pour objet l'utilisation d'un inhibiteur et/ou substrat de caspases, tel que le zVAD, pour la fabrication d'un médicament destiné à induire la mort de cellules indésirables. L'accélération de la mort cellulaire observée peut être une conséquence de la stabilisation de la PML mais peut également impliquer d'autres mécanismes, qui restent dans le cadre de la présente invention.

Par « substrat de caspases », on entend tout composé capable de se lier aux caspases.

Les auteurs de la présente invention ont également découvert que l'arsenic, et plus particulièrement le trioxyde d'arsenic, d'une part favorise l'adressage de la protéine PML vers les corps cellulaires et d'autre part accélère la mort cellulaire induite par les interférons.

La présente invention a plus particulièrement pour objet l'utilisation d'un composé de l'arsenic, à la condition que le composé de l'arsenic ne soit pas un composé de sulfure d'arsenic, ou d'un composé ayant les mêmes propriétés biologiques que l'arsenic pour la fabrication d'un médicament destiné à induire la mort de cellules indésirables, en association avec au moins un interféron.

Cette induction de la mort cellulaire observée peut être une conséquence de l'adressage de la protéine PML vers les corps cellulaires, mais peut également impliquer d'autres mécanismes, qui restent dans le cadre de la présente invention.

Parmi les composés de l'arsenic, on peut citer notamment le trioxyde d'arsenic ou le mélarsoprol.

Par « composé ayant les mêmes propriétés biologiques que l'arsenic », on entend tout composé qui, comme l'arsenic, est un inhibiteur de phosphatase et/ou est capable de créer des adduits covalents par liaison avec des groupements dithiols.

Les inhibiteurs et/ou substrats de caspases ou les composés de l'arsenic ou les composés ayant les mêmes propriétés biologiques que l'arsenic sont de préférence utilisés, pour induire la mort des cellules indésirables, en association avec la protéine PML, et/ou avec un agent induisant la surexpression de la protéine PML. Parmi les agents induisant la surexpression de la protéine PML, on utilise de préférence un interféron, tel que l'interféron α, β ou γ.

En effet les auteurs de la présente invention ont plus particulièrement découvert que les substrats de caspases, et en particulier le zVAD, ainsi que les composés de l'arsenic, en particulier le trioxyde d'arsenic, agissaient de manière synergique avec les interférons pour induire et accélérer la mort cellulaire.

L'administration de manière simultanée ou séquentielle de PML ou d'un agent induisant la surexpression de la protéine PML, tels que les interférons, peut être inutile si la quantité de PML ou d'agent induisant la surexpression de la protéine PML, tels que les interférons, d'origine endogène, est suffisante. Néanmoins, selon un mode préféré de réalisation de l'invention, l'administration d'une substance choisie parmi les composés de l'arsenic autres que les composés de sulfure d'arsenic, les composés ayant les mêmes propriétés biologiques que l'arsenic et les inhibiteurs et/ou substrats des caspases, est associée à une administration simultanée ou séquentielle de protéine PML, et/ou d'un agent induisant la surexpression de la protéine PML, tels que les interférons.

Fait partie de l'invention l'utilisation d'une substance choisie parmi les composés de l'arsenic autres que les composés de sulfure d'arsenic, les composés ayant les mêmes propriétés biologiques que l'arsenic et les inhibiteurs et/ou substrats des caspases, en association avec un interféron, pour induire la mort de cellules indésirables, que celle-ci soit médiée par la protéine PML induite par ledit interféron ou par un autre mécanisme également induit par ledit interféron.

La présente invention a également pour objet l'utilisation comme médicament d'au moins une substance choisie parmi les composés de l'arsenic autres que les composés de sulfure d'arsenic, les composés ayant les mêmes propriétés biologiques que l'arsenic et les inhibiteurs et/ou substrats des caspases, en association avec un véhicule pharmaceutiquement acceptable.

De manière préférentielle on peut administrer également audit patient une quantité thérapeutiquement efficace de protéine PML, et/ou d'un agent induisant la surexpression de la protéine PML, tel qu'un interféron, de manière simultanée ou séquentielle.

La présente invention a également pour objet une composition pharmaceutique contenant
1) soit au moins un inhibiteur et/ou substrat des caspases associé à :
   - au moins un composé de l'arsenic autre qu'un composé de surfure d'arsenic, ou un composé ayant les mêmes propriétés biologiques que l'arsenic;
   - et/ou la protéine PML
   - et/ou au moins un agent induisant la surexpression de la protéine PML, tel qu'un interféron;
      en présence d'un véhicule pharmaceutiquement acceptable ;
2) soit au moins un composé de l'arsenic ou un composé ayant les mêmes propriétés biologiques que l'arsenic autre qu'un composé de surfure d'arsenic, associé à la protéine PML et/ou à au moins un agent induisant la surexpression de la protéine PML, tel qu'un interféron, en présence d'un véhicule pharmaceutiquement acceptable.

La présente invention a également pour objet un kit comprenant
a)
   - une composition pharmaceutique (1) contenant au moins un inhibiteur et/ou substrat des caspases, en association avec un véhicule pharmaceutiquement acceptable ;
   - et/ou une composition pharmaceutique (2) contenant au moins un composé de l'arsenic autre qu'un composé de surfure d'arsenic, ou un composé ayant les mêmes propriétés que l'arsenic, en association avec un véhicule pharmaceutiquement acceptable ; et
b)
   - une composition pharmaceutique (3) contenant la protéine PML en association avec un véhicule pharmaceutiquement acceptable;
   - et/ou une composition pharmaceutique (4) contenant au moins un agent induisant la surexpression de la protéine PML, tel qu'un interféron, en association avec un véhicule pharmaceutiquement acceptable; lesdites compositions pharmaceutiques étant destinées à une administration simultanée ou séquentielle.

Le mode d'administration et la posologie dépendent de l'affection traitée et de son stade d'avancement, ainsi que du poids, de l'âge et du sexe du patient.

Conformément à l'invention, la formulation des médicaments de l'invention permet une administration notamment par voie orale, anale, nasale, intra-musculaire, intra-dermique, sous-cutanée, ou intra-veineuse.

La dose d'administration envisagée peut être par exemple de 1 à 50 mg par jour, de préférence par voie intraveineuse, pour les composés de l'arsenic, de 1 à 250 mg par kg de poids corporel de substrats de caspases tels que le zVAD, et de 1 à 20 millions d'unités internationales (M UI), de préférence de 3 à 5 M UI, de préférence par voie intra-musculaire ou sous-cutanée, par jour ou tous les deux jours, pour l'interféron.

Les auteurs de l'invention ont en outre découvert que la mort cellulaire induite par la surexpression de la protéine PML localisée sur les corps nucléaires présente des caractéristiques différentes de l'apoptose induit par les caspases. Dans le cas de la mort cellulaire induite par la PML, on n'observe en particulier pas les caractéristiques morphologiques nucléaires typiques de l'apoptose, telles que la condensation de la chromatine et la fragmentation nucléaire.

De plus, alors que la mort cellulaire induite par les interférons seuls présente les caractéristiques de l'apoptose, les auteurs de la présente invention ont observé que l'association synergique de zVAD avec les interférons fait disparaître ce phénotype apoptotique, la mort cellulaire présentant alors des caractéristiques différentes de celles de l'apoptose.

Une des conséquences majeures de cette découverte est la capacité des cellules indésirables tuées par le mécanisme induit par la PML de provoquer une réaction immunitaire contre des cellules indésirables similaires qui auraient échappé à la mort médiée par la protéine PML.

Cette propriété rend particulièrement intéressante l'utilisation d'une substance choisie parmi les composés de l'arsenic autres que les composés de sulfure d'arsenic, les composés ayant les mêmes propriétés biologiques que l'arsenic, et les inhibiteurs et/ou substrats des caspases, de préférence en combinaison avec un interféron, pour induire la mort de cellules indésirables, et/ou induire une réaction immunitaire.

Ainsi, l'administration de ces médicaments permettra une forme d'immunothérapie, ou de "vaccination", en provoquant une réaction du système immunitaire qui élimine les cellules indésirables survivantes, qu'il s'agisse de cellules cancéreuses, de cellules infectées, ou de toute autre cellule indésirable qui participe au développement d'une maladie.

Cette propriété est liée au fait que ces médicaments, en particulier l'association interféron et zVAD ou le zVAD seul, entraînent dans les cellules indésirables un phénomène de mort qui ne possède pas toutes les caractéristiques de l'apoptose, qui est le phénotype physiologique le plus fréquent du suicide cellulaire.

En particulier, l'inhibiteur de caspases zVAD empêche l'apparition de la plupart des manifestations de l'apoptose, qui dépendent d'une activation, dans la cellule mourante, de certaines caspases. En effet, si les caspases ne sont pas indispensables à l'exécution du suicide cellulaire, elles apparaissent en revanche indispensables au cours des phénomènes de suicide cellulaire, à l'exécution d'un phénotype de mort apoptotique (Xiang J. et al. PNAS 1996, 93 : 14559 ; Quignon F. et al. Nature Genetics 1998, 20 : 259 ; Vercammen D. et al, J. Exp. Med. 1998, 187 : 1477).

Un certain nombre de résultats suggèrent que la manière dont une cellule meurt joue un rôle important dans l'induction ou non d'une réaction immunitaire dirigée contre les cellules survivantes possédant les mêmes caractéristiques (y compris par exemple la nature de l'anomalie qui les rend cancéreuses, ou la nature de l'agent infectieux qu'elles contiennent).

La survenue dans une cellule d'un phénomène de mort apoptotique aurait pour effet de limiter l'induction d'une réaction immunitaire contre les cellules survivantes possédant les mêmes caractéristiques, voire de favoriser l'induction d'une forme de tolérance immunitaire, c'est à dire d'une inhibition sélective de l'induction d'une réponse immune dirigée contre les cellules survivantes possédant les mêmes caractéristiques. Des résultats obtenus dans certains modèles particuliers (*in vitro,* ou *in vivo* dans la chambre antérieure de l'oeil de la souris), suggèrent que la mort apoptotique d'une cellule pourrait d'une manière générale limiter ou inhiber l'induction des réactions d'immunité à médiation cellulaire de type hypersensibilité retardée (dites Th1) médiées par les lymphocytes T CD4+, (Griffith T. et al, Immunity 1996, 5 : 7 ; Voll R. et al, Nature 1997, 390 : 350 ; Gao Y. et al., J. Exp. Med. 1998, 188 : 887), réaction qui représente l'une des manifestations essentielles d'une réaction immune efficace contre des cellules indésirables.

Dans un modèle particulier de tumeurs chez la souris, il a été montré que la survenue dans des cellules cancéreuses de phénomènes de mort apoptotique n'entraîne pas l'induction d'une réaction immunitaire efficace dirigée contre les cellules cancéreuses vivantes présentant les mêmes caractéristiques, alors que l'introduction artificielle dans ces cellules cancéreuses d'un gène qui empêche la survenue dans les cellules cancéreuses mourantes d'une partie du phénotype apoptotique, permet l'induction d'une réaction immunitaire efficace dirigée contre les cellules cancéreuses vivantes présentant les mêmes caractéristiques mais dans lesquelles ce gène n'a pas été artificiellement introduit (Melcher et al, 1998,Nature Med., vol. 4, n° 5, pp 581-587).

La découverte que les médicaments décrits dans cette demande provoque la mort de cellules indésirables tout en n'entraînant pas (ou en empêchant) la survenue dans les cellules mourantes d'un phénotype apoptotique est donc importante. Elle implique que ces médicaments auront pour effet non seulement de provoquer la mort des cellules indésirables, mais aussi de permettre l'induction concomitante d'une réaction immunitaire efficace permettant l'élimination concomitante ou ultérieure des cellules indésirables qui auraient échappé à la mort induite par les médicaments.

Cette propriété peut également être mise à profit pour traiter *ex vivo* un ensemble de cellules susceptibles de contenir des cellules indésirables, avant administration à un patient, par exemple une préparation de moelle osseuse destinée à une greffe chez un patient leucémique, une telle préparation contenant généralement quelques cellules malignes résiduelles. Un tel traitement permet non seulement d'induire la mort des cellules indésirables contenues dans la préparation, mais encore de provoquer une réaction immunitaire dirigée contre les cellules indésirables présentes dans le corps du patient à qui la préparation cellulaire traitée est administrée.

La présente invention a donc également pour objet un procédé *in vitro* pour induire la mort de cellules indésirables comprenant la mise en contact de cellules indésirables avec une substance choisie parmi les composés de l'arsenic autres que les composés de sulfure d'arsenic, les composés ayant les mêmes propriétés biologiques que l'arsenic, et les inhibiteurs et/ou substrats des caspases, ladite substance pouvant être de préférence associée à la protéine PML et/ou à un agent induisant la surexpression de la protéine PML, de préférence un interféron.

Les exemples et figures suivants illustrent l'invention sans en limiter la portée.

### LEGENDE DES FIGURES

- La figure 1A représente l'induction de la protéine PML de 90 kD dans un clone REF(T)PML, quatre heures après exposition à des concentrations variables de ZnCl₂.
- La figure 1B représente une analyse par FACS de cellules REF(T)PML ou de cellules contrôle, quatre heures 30 après exposition à 150 µM de ZnCl₂. Le panneau de gauche représente le contenu en ADN par rapport à la taille des cellules. Les panneaux de droite représente le contenu en ADN en fonction de la fluorescence (TUNEL).
- La figure 1C représente l'analyse cytométrique des cellules REF(T)PML traitées ou non avec du ZnCl₂, de l'étoposide, ou l'inhibiteur de caspase zVAD. Un marquage est effectué avec de l'Annexine V-FITC (panneau de gauche) ou de la Rhodamine 123 (panneau de droite). Le pourcentage en cellules apoptotiques est indiqué.
- La figure 2A représente l'absence de clivage de la PARP lors de la mort cellulaire déclenchée par la PML. Les cellules ont été traitées avec 150 µM de ZnCl₂, de l'étoposide ou du zVAD.
- La figure 2B représente l'activité de la caspase CPP32 déterminée par coupure du DEVD-pNA dans des cellules REF(T) contrôle ou des cellules REF(T)PML. Les absorbances relatives de trois déterminations indépendantes sont présentées.
- La figure 3A montre la survie des monocytes traités avec 1000 U/ml d'IFNα. Une expérience représentative sur cinq est présentée. Les tests TUNEL démontrent que la diminution de la numération cellulaire est due à l'époptose.
- La figure 3B représente des histogrammes indiquant l'effet du zVAD (100 µM) 24 heures après l'addition de celui-ci. Les valeurs moyennes ± les écarts-type de 11 expériences sont présentées.
- La figure 4A montre que le zVAD stabilise la protéine PML dans les cellules REF(T).
- La figure 4B montre que l'interféron α (1 000 U/ml) induit la PML de rat dans les cellules REF(T)PML. Les flèches indiquent des isoformes distincts de la PML.

### EXEMPLES

### MATERIELS ET METHODES

### - Construction plasmidique

Un fragment *SacI-Bgll*I (- 69, + 55 paires de base) du promoteur de la métallothionéine de souris a été inséré dans un plasmide pKS et a été fusionné avec un fragment *Bgl*II-*Bam*HI d'un ADNc de PML conduisant au plasmide pKSmMT-PML. Pour la fusion GFP-PML, le même fragment PML a été inséré dans le site *Bgl*II du vecteur pEGFP-1 (Clontech). Un vecteur rétroviral exprimant PML a été également construit par insertion d'un ADNc de pleine longueur de PML (de Thé et al, 1991) au site *Eco*RI de SRαtkneo (Muller et al, 1991).

### - Culture cellulaire

Les cellules REF(T) et MEF(T) sont des fibroblastes embryonnaires de rats et de souris immortalisées par un vecteur d'expression SV40T. Les cellules REF(K1) sont immortalisées par un mutant SV40T qui ne lie pas Rb, et les cellules F111 sont des fibroblastes de rat 3T3 spontanément immortalisés. Pour des essais de clonogénicité, les cellules ont été transfectées avec 10 µg de SRαtkneo-PML ou SRαtkneo sur des boîtes de culture de 10 cm de diamètre et sélectionnées par de la néomycine (500 µg/ml). Pour obtenir des clones inductibles, un pool de cellules REF(T) a été co-transfecté avec le plasmide pKSmMt-PML et un vecteur de résistance à l'hygromycine (DSP-Hygro). Les colonies résistantes ont été examinées pour l'expression de la PML après quatre heures d'un traitement au ZnCl₂ (150 µM) et soumises à un deuxième cycle de clonage par dilution limite. Des clones CHO inductible ont été construits de manière similaire. Les monocytes ont été préparés selon la méthode d'Estaquier et al, 1997. L'étoposide (utilisé à 100 µM pendant 16 à 24 heures) a été obtenu auprès de Biomol Research Laboratories, le zVAD (benzyloxycarbonyl-Val-Ala-Asp fluorométhylcétone, utilisé à 250 µg/ml) est commercialisé chez Bachem et l'IFNα de rat chez Access BioMedical. L'interféron α humain a été fourni par Schering-Plough. Les anticorps contre la protéine PML humaine sont décrits dans l'article de Daniel et al, 1993. Les expériences de Western Blot avec la protéine PML de rat endogène ont été réalisées avec l'anticorps monoclonal 5E10 qui détecte à la fois la PML de rat et la PML humaine.

### - Evaluation de la mort cellulaire

Les cellules ont été traitées pendant 2 heures avec 150 µM de ZnCl₂ (sauf indications contraires) en présence ou l'absence de sérum de veau foetal inactivé par la chaleur, puis les cellules ont été lavées et incubées dans un milieu sans ZnCl₂. Le test TUNEL a été réalisé selon les indications du fabricant (Boehringer Mannheim, kit de détection de la mort cellulaire *in situ),* à l'exception de l'étape de fixation (4 % de formaldéhyde dans du tampon phosphate EBS pendant 10 minutes). La teneur en ADN cellulaire a été évaluée par une incubation de 10 minutes dans de l'iodure de propidium à 50 µg/ml, en présence de Rnase A à 100 µg/ml à 4°C. L'analyse de l'expression de la phosphatidylsérine sur le feuillet externe des membranes cellulaires a été réalisée en utilisant un marquage Annexine-V-fluos (Boehringer Mannheim) et une perte de la polarité mitochondriale par Rhodamine 123 (Molecular probes) selon les instructions du fabricant. Les échantillons ont été analysés sur un analyseur FACScan (Lysis II software Becton Dickinson). Pour le clivage de substrat par les caspases, 5.10⁶ cellules ont été lavées dans du tampon PBS, et incubées pendant une heure à 4°C dans 200 µl de tampon de lyse (10 mM Hepes, pH 7,4, 2 mM EDTA, 2 mM DTT, 0,1 % CHAPS). Après centrifugation, 20 µl de surnageant et 180 µl de tampon de réaction (100 mM Hepes, pH 7,4, 2 % de glycérol, 5 mM DTT, 0,5 mM EDTA, 50 µM DEVD-pNA (Biomol Research Laboratories)) ont été mélangés et l'absorbance à 405 nm a été mesurée après quatre heures d'incubation à 37°C. L'anticorps polyclonal SA-252 anti-PARP est commercialisé chez Biomol Research Laboratories.

### EXEMPLE 1 :

### La PML induit une mort cellulaire indépendante du zVAD.

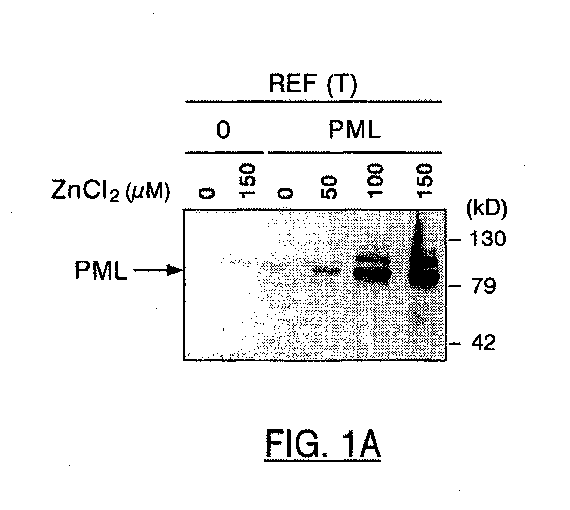

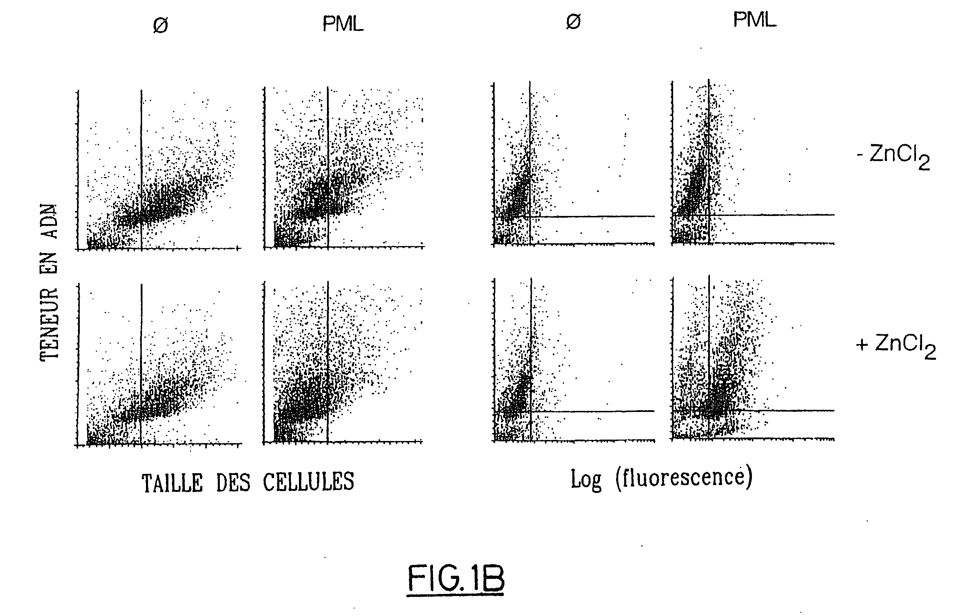

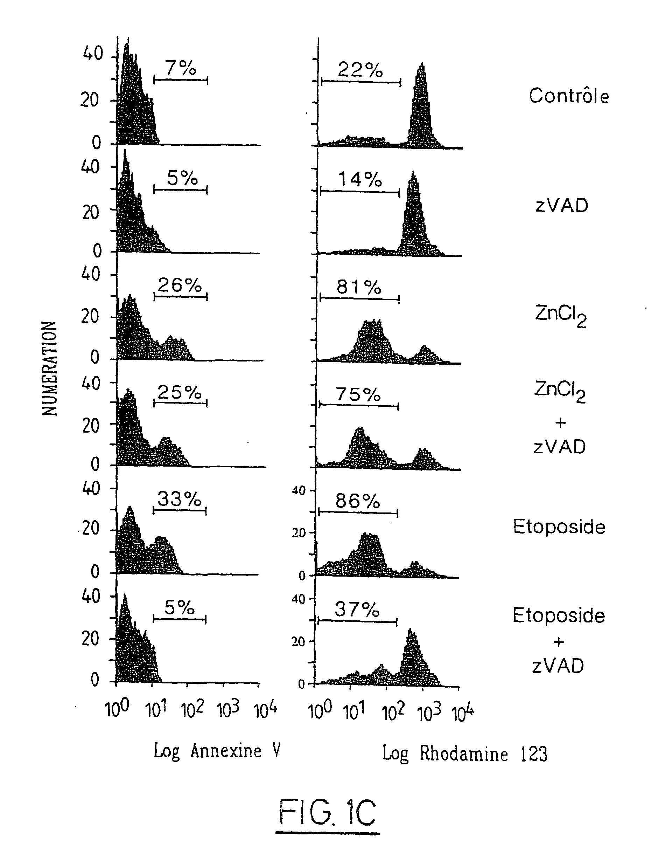

La transfection d'un vecteur d'expression de la PML (pSG5-PML) dans différentes lignées cellulaires de fibroblastes a fortement diminué la formation de foyers. La protéine PML étant indétectable dans les clones obtenus à partir des cellules transfectées par la PML, ces résultats impliquent que la PML exerce un effet inhibiteur majeur soit sur le cycle cellulaire soit sur la survie des cellules. Pour comprendre le mécanisme à la base de cet effet, un pool de fibroblastes d'embryons de rat (REFs) transformés par SV40T a été transfecté avec un plasmide pKSmMT-PML, dans lequel l'expression de PML est sous le contrôle d'un promoteur de la métallothionéine de souris. Trois des clones résultant REF(T)PML ont été étudiés par la suite, tandis que trois clones REF(T) porteurs du vecteur vide ont été testés comme contrôle. La protéine PML a été détectée par Western Blot deux heures après exposition au ZnCl₂ (expression détectable à partir de 50 µM de ZnCl₂ et présentant un plateau à 150 µM de ZnCl₂) (figure 1A). L'expression de la PML a induit une mort cellulaire synchronisée de l'ensemble de la population cellulaire avec des cinétiques variant de 48 heures pour 50 µM de Zn Cl₂ à six heures pour 150 µM. Dans les trois clones REF(T)PML, des modifications morphologiques ont été observées à partir de trois heures après induction avec 150 µM de ZnCl₂. Les cellules s'arrondissent, avec un rétrécissement clair du cytoplasme (figure 1B), sont devenus positives dans un test TUNEL (figure 1B) puis se sont progressivement détachées de la boîte. Elles ont cependant gardé leur capacité à exclure le bleu trypan. Ces modifications ont été associées avec une teneur en ADN subG1 modeste (figure 1B), une externalisation de la phosphatidylserine membranaire (figure 1C) et une perte du potentiel transmembranaire mitochondrial (figure 1C). Tandis que des modifications similaires ont été observées dans l'apoptose induite par l'agent génotoxique étoposide, elles n'ont jamais été trouvées dans les cellules contrôle REF(T) traitées avec ZnCl₂ (figures 1B et C). Contrairement au traitement par l'étoposide, la mort cellulaire induite par la PML n'est pas associée avec des caractéristiques morphologiques nucléaires typiques de l'apoptose telles que la condensation de la chromatine et la fragmentation nucléaire, même tardives dans le processus de mort cellulaire. Malgré le clivage de l'ADN (sub-G1 positif (figure 1B) et perte de la viscosité de l'ADN), la mort cellulaire induite par la PML n'est pas associée avec une échelle de l'ADN internucléosomale, en conformité avec le faible signal positif du TUNEL (figure 1B).

### Contrôles :

Comme les cellules REF(T) sont des lignées cellulaires transformées par SV40T, plusieurs expériences ont été réalisées pour exclure une contribution de l'oncogène « grand T » du virus SV40 à la mort cellulaire induite par la PML. Premièrement, l'expression de la PML n'a pas altéré l'expression ou la localisation de SV40T dans les cellules REF(T)PML ni n'a dégradé la p53 ou la libération de p53 à partir de l'oncogène « grand T » du virus SV40. Deuxièmement, dans les cellules HeLa ou CHO transfectées de manière transitoire avec soit une protéine de fusion GFP-PML ou du GFP seul, toutes les cellules GFP-PML positives se sont progressivement détachées de la boîte et sont mortes, contrairement aux cellules GFP positives contrôle. Troisièmement, dans les cellules CHO transfectées de manière stable avec le plasmide pKSmMT-PML, l'induction par le ZnCl₂ a là encore conduit à la mort des clones exprimant la protéine PML. Enfin, dans les cellules REF exprimant un mutant thermosensible SV40T, la dégradation des SV40T à 39,5°C n'a pas affecté la mort cellulaire déclenchée par la PML.

L'induction de la mort cellulaire peut requérir une transcription de novo ou peut refléter le déclenchement de voies préexistantes. Les cellules REF(T)PML ont été tout d'abord incubées avec ZnCl₂ et avec du cycloheximide pendant deux heures permettant ainsi la synthèse d'ARNm de PML, et non pas sa traduction. Les cellules ont ensuite été lavées et incubées avec de l'actinomycine D seule pour permettre la traduction de l'ARNm de PML mais pas la néosynthèse ARNm. Dans cette expérience, la mort cellulaire a été observée de même qu'en absence d'inhibiteur, montrant que la transcription de novo n'est pas requise. Une mort induite par la PML ne nécessite pas et n'induit pas la transition vers la phase S du cycle cellulaire. En effet, la PML déclenche toujours la mort dans les cellules REF(T)PML qui ont été bloquées au stade G1/S par un traitement à l'aphidicoline. De plus, une exposition au BrdU à différents temps après induction par ZnCl₂ a montré que la réplication de l'ADN n'était pas modifiée jusqu'à deux heures mais était arrêtée après trois heures et que la mort cellulaire était présente dans toutes les phases du cycle cellulaire (figure 1B).

### EXEMPLE 2 :

### L'arsenic favorise la mort cellulaire déclenchée par la PML.

Lorsque les cellules REF(T)PML ont été traitées avec du ZnCl₂ et 10⁻⁶ M d'As₂O₃, une vive accélération dans les modifications morphologiques associées à la mort cellulaire a été observée. Le clivage de l'ADN déterminé par des tests TUNEL a augmenté de manière similaire (117 % de cellules positives pour le co-traitement avec ZnCl₂ contre 45 % pour ZnCl₂ seul, tandis que As₂O₃ seul n'a induit aucune augmentation par rapport au niveau de base). Le fait que l'arsenic augmente l'induction de la mort cellulaire en parallèle à la localisation de la PML sur les corps nucléaires suggère que la localisation de la PML vers les corps nucléaires est importante pour la mort cellulaire.

### EXEMPLE 3 :

### La mort déclenchée par la PML n'est pas associée à l'activation des caspases.

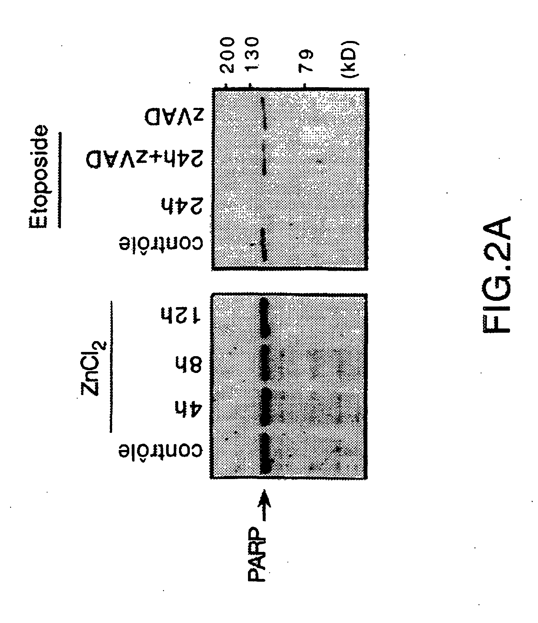

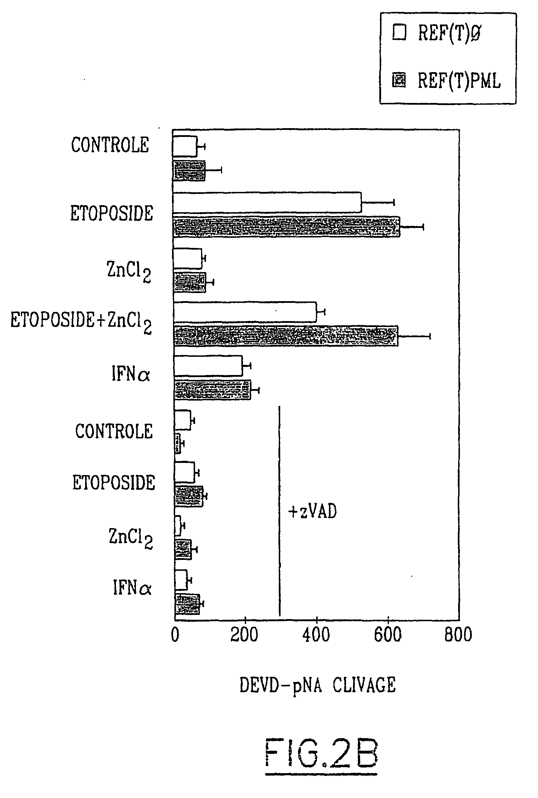

Il est connu que l'exécution de la mort cellulaire programmée implique l'activation protéolytique de caspases qui induisent les changements phénotypiques de l'apoptose par clivage de protéines nucléaires et cytoplasmiques (Salvesen et al, 1997). L'inhibiteur de caspases zVAD qui bloque l'apoptose induite par l'étoposide, n'inhibe pas la mort cellulaire induite par la
PML (figure 1C) et même de manière paradoxale l'accélère (71 % de signal positif TUNEL avec du zVAD et du ZnCl₂ contre 45 % avec du ZnCl₂ seul). Ces observations impliquent que les exécutants sensibles à la zVAD ne sont pas requis pour la mort cellulaire induite par la PML. De plus, la CPP32 (caspase 3), la principale caspase impliquée dans l'apoptose, apparaît ne pas être activée durant la mort cellulaire induite par la PML, dans la mesure où, un de ses substrats, la PARP (poly(ADP-ribose)polymérase) reste non clivé (figure 2A). Contrairement à l'étoposide, aucun clivage significatif des substrats de la caspase colorimétrique, YVAD-pNa (caspase de classe 1, Boeringher Mannheim) et DEVD-pNA (caspase de classe 3, Boeringher Mannheim) après induction par la PML n'a pu être détecté (figure 2B).

### EXEMPLE 4 :

### L'arsenic et le zVAD potentialisent la mort cellulaire induite par la PML et les interférons.

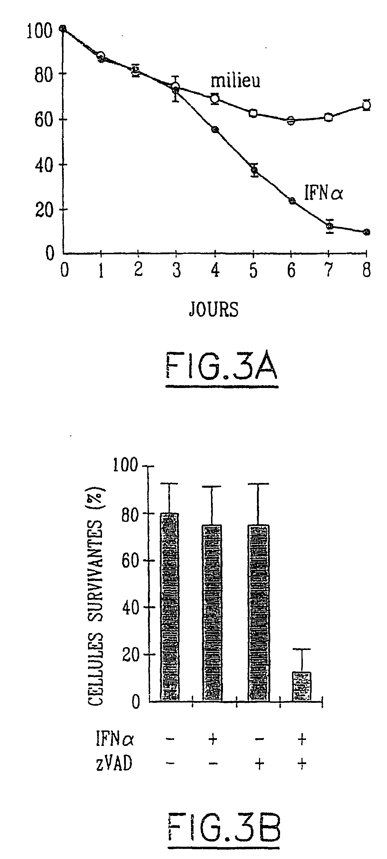

Des monocytes primaires exposés à l'interféron α ont subi une mort cellulaire progressive qui a conduit à la disparition complète de la culture cellulaire après sept jours (figures 3A et 3B). Lors de l'addition de zVAD avec l'interféron α, la mort de l'ensemble de la population cellulaire a été observée dans les 24 heures en l'absence de la fragmentation nucléaire et de la condensation de la chromatine observée avec l'interféron seul (figures 3A et 3B). Peu ou aucune mort cellulaire n'a été observée avec du zVAD seul pendant 20 jours dans la plupart des cultures primaires (8/11) (figures 3A et 3B). Dans trois cultures sur onze, le zVAD seul a induit la mort d'une partie de la culture après sept jours, ces résultats reflétant probablement une sécrétion endogène d'interféron. Des résultats similaires ont été obtenus avec d'autres inhibiteurs de caspase tel que le DEVD.

Le tableau ci-dessous représente la mort cellulaire évaluée par tunnel de cellules REF(T)PML traitées pendant deux jours avec 1 000 U/ml d'IFNα et 10⁻⁶ M d'As₂O₃ ou de zVAD.

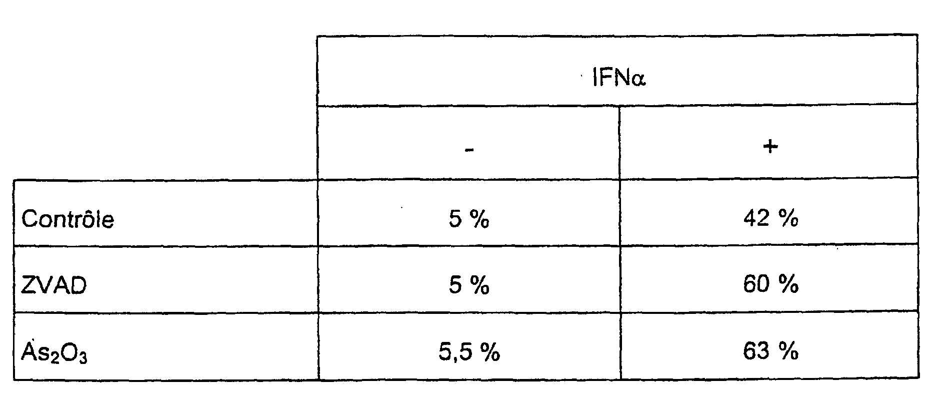

Dans les cellules REF(T), une synergie importante a été trouvée entre soit l'interféron α et le zVAD, soit l'interféron α et l'As₂O₃ (42 % de signal positif TUNEL pour l'interféron α seul, et 60 % et 63 % avec zVAD et arsenic respectivement).

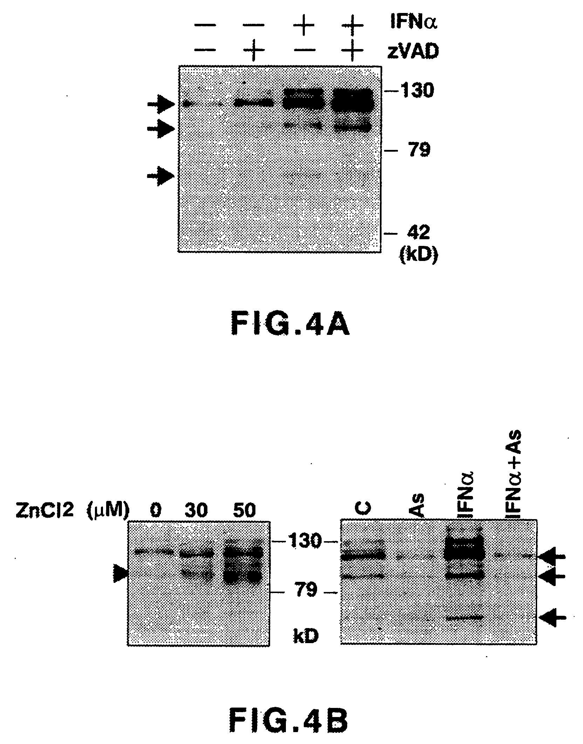

Le zVAD a augmenté les niveaux d'expression de la PML (figure 4A) et l'arsenic a augmenté son association avec les corps nucléaires, alors que la quantité totale en PML a été diminuée. La similarité de la synergie du zVAD et de l'arsenic avec les morts cellulaires déclenchées par la PML et l'interféron suggère que la PML est impliquée la mort cellulaire induite par l'interféron. De plus, l'interféron α induit la mort cellulaire avec les mêmes cinétiques que le ZnCl₂ 50 µM et les deux induisent des quantités similaires de protéine PML (figure 4B).

## Revendications

1. Combinaison d'au moins une substance:
- qui favorise l'adressage de la protéine PML vers les corps nucléaires et/ou sa stabilisation; et/ou
- qui est choisie parmi les composés de l'arsenic, à la condition que les composés de l'arsenic ne soient pas des composés de sulfure d'arsenic, les composés ayant les mêmes propriétés biologiques que l'arsenic, et les inhibiteurs et/ou substrats des caspases,
avec la protéine PML et/ou un agent induisant la surexpression de la protéine PML pour utilisation comme médicament.

2. Utilisation d'au moins une substance:
- qui favorise l'adressage de la protéine PML vers les corps nucléaires et/ou sa stabilisation; et/ou
- qui est choisie parmi les composés de l'arsenic, à la condition que les composés de l'arsenic ne soient pas des composés de sulfure d'arsenic, les composés ayant les mêmes propriétés biologiques que l'arsenic, et les inhibiteurs et/ou substrats des caspases,
en combinaison avec la protéine PML et/ou à un agent induisant la surexpression de la protéine PML;
pour la fabrication d'un médicament destiné à induire la mort de cellules indésirables et/ou stimuler une réaction immunitaire, l'administration de ladite substance et l'administration de la protéine PML et/ou dudit agent induisant la surexpression de la protéine PML étant simultanées ou séquentielles.

3. Utilisation selon la revendication 2, dans laquelle les cellules indésirables sont choisies parmi les cellules d'une tumeur, les cellules infectées par un virus, un parasite ou une bactérie, des cellules immunitaires participant à une réaction immunitaire inappropropriée, des cellules génétiquement altérées, des cellules sénescentes ou hyperplasiques.

4. Utilisation d'au moins une substance:
- qui favorise l'adressage de la protéine PML vers les corps nucléaires et/ou sa stabilisation; et/ou
- qui est choisie parmi les composés de l'arsenic, à la condition que les composés de l'arsenic ne soient pas des composés de sulfure d'arsenic, les composés ayant les mêmes propriétés biologiques que l'arsenic, et les inhibiteurs et/ou substrats des caspases,
en combinaison avec la protéine PML et/ou à un agent induisant la surexpression de la protéine PML;
pour la fabrication d'un médicament destiné au traitement d'une tumeur.

5. Utilisation selon la revendication 4, dans laquelle ladite tumeur est choisi parmi les cancers solides, les leucémies et lymphomes.

6. Utilisation selon la revendication 4 ou 5, dans laquelle ladite tumeur est choisi parmi les leucémies lymphoides T de l'adulte (ALT) et les mélanomes.

7. Utilisation selon l'une quelconque des revendications 1 à 6, dans laquelle ladite substance est le trioxyde d'arsenic.

8. Utilisation selon l'une quelconque des revendications 1 à 6, dans laquelle ladite substance est le mélarsoprol.

9. Utilisation selon l'une quelconque des revendications 1 à 6, dans laquelle ladite substance est le zVAD.

10. Utilisation selon l'une quelconque des revendications 1 à 9, dans laquelle ledit agent induisant la surexpression de la protéine PML est un interféron, tel que l'interféron α, β ou γ.

11. Utilisation selon la revendication 10, dans laquelle ledit agent induisant la surexpression de la protéine PML est l'interféron α.

12. Utilisation selon la revendication 10, dans laquelle ledit agent induisant la surexpression de la protéine PML est l'interféron β.

13. Utilisation selon la revendication 10, dans laquelle ledit agent induisant la surexpression de la protéine PML est l'interféron γ.

14. Utilisation d'au moins une substance choisie parmi les composés de l'arsenic, à la condition que les composés de l'arsenic ne soient pas des composés de sulfure d'arsenic, les composés ayant les mêmes propriétés biologiques que l'arsenic,et les inhibiteurs et/ou substrats des caspases, en combinaison avec un interféron, pour la fabrication d'un médicament destiné à induire la mort de cellules indésirables et/ou stimuler une réaction immunitaire, l'administration de ladite substance et l'administration de l'interféron étant simultanées ou séquentielles.

15. Procédé in vitro pour induire la mort de cellules indésirables comprenant la mise en contact de cellules indésirables avec une substance choisie parmi les composés de l'arsenic, les composés ayant les mêmes propriétés biologiques que l'arsenic, et les inhibiteurs et/ou substrats des caspases, ladite substance étant associée à la protéine PML et/ou à un agent induisant la surexpression de la protéine PML, de préférence un interféron.

16. Composition pharmaceutique contenant
a) soit au moins un inhibiteur et/ou substrat des caspases associé à:
- au moins un composé de l'arsenic ou un composé ayant les mêmes propriétés biologiques que l'arsenic;
- et/ou la protéine PML;
- et/ou au moins un agent induisant la surexpression de la protéine PML, tel qu'un interféron;
en présence d'un véhicule pharmaceutiquement acceptable;
b) soit au moins un composé de l'arsenic, à la condition que le composé de l'arsenic ne soit pas un composé de sulfure d'arsenic, ou un composé ayant les mêmes propriétés biologiques que l'arsenic associé à la protéine PML et/ou à au moins un agent induisant la surexpression de la protéine PML, tel qu'un interféron, en présence d'un véhicule pharmaceutiquement acceptable.

17. Kit comprenant
a)
- une composition pharmaceutique (1) contenant au moins un inhibiteur et/ou substrat des caspases, en association avec un véhicule pharmaceutiquement acceptable;
- et/ou une composition pharmaceutique (2) contenant au moins un composé de l'arsenic, à la condition que le composé de l'arsenic ne soit pas un composé de sulfure d' arsenic, ou un composé ayant les mêmes propriétés que l'arsenic, en association avec un véhicule pharmaceutiquement acceptable; et
b)
- une composition pharmaceutique (3) contenant la protéine PML en association avec un véhicule pharmaceutiquement acceptable;
- et/ou une composition pharmaceutique (4) contenant au moins un agent induisant la surexpression de la protéine PML, tel qu'un interféron, en association avec un véhicule pharmaceutiquement acceptable;
lesdites compositions pharmaceutiques étant destinées à une administration simultanée ou séquentielle.

18. Utilisation d'au moins une substance, autre que les composés de l'arsenic, favorisant l'adressage de la protéine PML vers les corps nucléaires et/ou sa stabilisation, pour la fabrication d'un médicament destiné à induire la mort de cellules indésirables, et/ou stimuler une réaction immunitaire.

19. Utilisation selon la revendication 18, dans laquelle ladite substance est choisie parmi les inhibiteurs et/ou substrats des caspases.

20. Utilisation selon la revendication 15 dans laquelle laditesubstance est le zVAD.

## Patentansprüche

1. Kombination aus mindestens einer Substanz:
- welche die Adressierung des PML-Proteins zu den Kernkörperchen und/oder seine Stabilisierung fördert und/oder
- welche aus Arsenverbindungen, mit der Maßgabe, daß die Arsenverbindungen keine Arsensulfidverbindungen sind, Verbindungen, welche die gleichen biologischen Eigenschaften wie Arsen aufweisen, und Inhibitoren und/oder Substraten von Caspasen ausgewählt ist,
mit dem PML-Protein und/oder einem Mittel, das die Überexpression des PML-Proteins induziert, zur Verwendung als Medikament.

2. Verwendung mindestens einer Substanz:
- welche die Adressierung des PML-Proteins zu den Kernkörperchen und/oder seine Stabilisierung fordert, und/oder
- welche aus Arsenverbindungen, mit der Maßgabe, daß die Arsenverbindungen keine Arsensulfidverbindungen sind, Verbindungen, welche die gleichen biologischen Eigenschaften wie Arsen aufweisen, und Inhibitoren und/oder Substraten von Caspasen ausgewählt ist,
in Kombination mit dem PML-Protein und/oder einem Mittel, das die Überexpression des PML-Proteins induziert,
zur Herstellung eines Medikaments zur Induzierung des Todes von unerwünschten Zellen und/oder zur Stimulierung einer Immunreaktion, wobei die Verabreichung der Substanz und die Verabreichung des PML-Proteins und/oder des die Überexpression des PML-Proteins induzierenden Mittels gleichzeitig oder aufeinanderfolgend erfolgt.

3. Verwendung nach Anspruch 2, wobei die unerwünschten Zellen aus Zellen eines Tumors, Zellen, die durch einen Virus, einen Parasiten oder ein Bakterium infiziert sind, Immunzellen, die an einer unangemessenen Immunreaktion teilhaben, genetisch veränderten Zellen, gealterten oder hyperplastischen Zellen ausgewählt sind.

4. Verwendung mindestens einer Substanz:
- welche die Adressierung des PML-Proteins zu den Kernkörperchen und/oder seine Stabilisierung fördert, und/oder
- welche aus Arsenverbindungen, mit der Maßgabe, daß die Arsenverbindungen keine Arsensulfidverbindungen sind, Verbindungen, welche die gleichen biologischen Eigenschaften wie Arsen aufweisen, und Inhibitoren und/oder Substraten von Caspasen ausgewählt ist,
in Kombination mit dem PML-Protein und/oder einem Mittel, das die Überexpression des PML-Proteins induziert,
zur Herstellung eines Medikaments zur Behandlung eines Tumors.

5. Verwendung nach Anspruch 4, wobei der Tumor aus soliden Tumoren, Leukämien und Lymphomen ausgewählt ist.

6. Verwendung nach Anspruch 4 oder 5, wobei der Tumor aus Lymphoid-T-Leukämien der Erwachsenen (ALT) und Melanomen ausgewählt ist.

7. Verwendung nach einem der Ansprüche 1 bis 6, wobei die Substanz Arsentrioxid ist.

8. Verwendung nach einem der Ansprüche 1 bis 6, wobei die Substanz Melarsoprol ist.

9. Verwendung nach einem der Ansprüche 1 bis 6, wobei die Substanz zVAD ist.

10. Verwendung nach einem der Ansprüche 1 bis 9, wobei das die Überexpression des PML-Proteins induzierende Mittel ein Interferon, wie Interferon-α, -β oder -γ ist.

11. Verwendung nach Anspruch 10, wobei das die Überexpression des PML-Proteins induzierende Mittel Interferon-α ist.

12. Verwendung nach Anspruch 10, wobei das die Überexpression des PML-Proteins induzierende Mittel Interferon-β ist.

13. Verwendung nach Anspruch 10, wobei das die Überexpression des Proteins PML induzierende Mittel Interferon-γ ist.

14. Verwendung mindestens einer Substanz, ausgewählt aus Arsenverbindungen, mit der Maßgabe, daß die Arsenverbindungen keine Arsensulfidverbindungen sind, Verbindungen, welche die gleichen biologischen Eigenschaften wie Arsen aufweisen, und Inhibitoren und/oder Substraten von Caspasen, in Kombination mit einem Interferon zur Herstellung eines Medikaments zur Induzierung des Todes von unerwünschten Zellen und/oder zur Stimulierung einer Immunreaktion, wobei die Verabreichung der Substanz und die Verabreichung des Interferons gleichzeitig oder aufeinanderfolgend erfolgt.

15. *In vitro*-Verfahren zur Induzierung des Todes von unerwünschten Zellen, umfassend das Inkontaktbringen von unerwünschten Zellen mit einer Substanz, ausgewählt aus Arsenverbindungen, Verbindungen, welche die gleichen biologischen Eigenschaften wie Arsen aufweisen, und Inhibitoren und/oder Substraten von Caspasen, wobei die Substanz mit dem PML-Protein und/oder einem die Überexpression des PML-Proteins induzierenden Mittel, vorzugsweise einem Interferon, assoziiert ist.

16. Pharmazeutische Zusammensetzung, enthaltend
a) entweder mindestens einen Inhibitor und/oder ein Substrat von Caspasen, assoziiert mit:
- mindestens einer Arsenverbindung oder einer Verbindung, welche die gleichen biologischen Eigenschaften wie Arsen aufweist,
- und/oder dem PML-Protein,
- und/oder mindestens einem die Überexpression des PML-Proteins induzierenden Mittel, wie Interferon,
in Gegenwart eines pharmazeutisch verträglichen Vehikels,
b) oder mindestens eine Arsenverbindung, mit der Maßgabe, daß die Arsenverbindung keine Arsensulfidverbindung ist, oder eine Verbindung, welche die gleichen biologischen Eigenschaften wie Arsen aufweist, assoziiert mit dem PML-Protein und/oder mit mindestens einem die Überexpression des PML-Proteins induzierenden Mittel, wie Interferon, in Gegenwart eines pharmazeutisch verträgliche Vehikels.

17. Kit, enthaltend
a)
- eine pharmazeutische Zusammensetzung (1), enthaltend mindestens einen Inhibitor und/oder ein Substrat von Caspasen, in Verbindung mit einem pharmazeutisch verträglichen Vehikel,
- und/oder eine pharmazeutische Zusammensetzung (2), enthaltend mindestens eine Arsenverbindung, mit der Maßgabe, daß die Arsenverbindung keine Arsensulfidverbindung ist, oder eine Verbindung, welche die gleichen Eigenschaften wie Arsen aufweist, in Verbindung mit einem pharmazeuisch verträglichen Vehikel, und
b)
- eine pharmazeutische Zusammensetzung (3), enthaltend das PML-Protein in Verbindung mit einem pharmazeutisch verträglichen Vehikel,
- und/oder eine pharmazeutische Zusammensetzung (4), enthaltend mindestens ein die Überexpression des PML-Proteins induzierendes Mittel, wie Interferon, in Verbindung mit einem pharmazeutisch verträglichen Vehikel,
wobei die pharmazeutischen Zusammensetzungen zur gleichzeitigen oder aufeinanderfolgenden Verabreichung bestimmt sind.

18. Verwendung mindestens einer Substanz, die keine Arsenverbindung ist, welche die Adressierung des PML-Proteins zu den Kernkörperchen und/oder seine Stabilisierung fördert, zur Herstellung eines Medikaments zur Induzierung des Todes unerwünschter Zellen und/oder zur Stimulierung einer Immunreaktion.

19. Verwendung nach Anspruch 18, wobei die Substanz aus Inhibitoren und/oder Substraten von Caspasen ausgewählt ist.

20. Verwendung nach Anspruch 15, wobei die Substanz zVAD ist.

## Claims

1. Combination of at least one substance:
- which promotes the targeting of the protein PML towards the nuclear bodies and/or the stabilisation thereof; and/or
- which is selected from the arsenic compounds, provided that the arsenic compounds are not arsenic sulphide compounds, compounds having the same biological properties as arsenic, and inhibitors and/or substrates of caspases,
with the protein PML and/or an agent which induces overexpression of the protein PML, for use as a medicament.

2. Use of at least one substance:
- which promotes the targeting of the protein PML towards the nuclear bodies and/or the stabilisation thereof; and/or
- which is selected from the arsenic compounds, provided that the arsenic compounds are not arsenic sulphide compounds, compounds having the same biological properties as arsenic, and inhibitors and/or substrates of caspases,
combined with the protein PML and/or an agent which induces overexpression of the protein PML;
for the production of a medicament intended to induce the death of undesirable cells and/or stimulate an immune reaction, the administration of said substance and the administration of the protein PML and/or of said agent which induces overexpression of the protein PML being simultaneous or sequential.

3. Use according to claim 2, wherein the undesirable cells are selected from among the cells of a tumour, cells infected with a virus, a parasite or a bacterium, immune cells participating in an inappropriate immune reaction, genetically modified cells, ageing or hyperplasic cells.

4. Use of at least one substance:
- which promotes the targeting of the protein PML towards the nuclear bodies and/or the stabilisation thereof; and/or
- which is selected from the arsenic compounds, provided that the arsenic compounds are not arsenic sulphide compounds, compounds having the same biological properties as arsenic, and inhibitors and/or substrates of caspases,
combined with the protein PML and/or an agent which induces overexpression of the protein PML;
for the production of a medicament intended for treating a tumour.

5. Use according to claim 4, wherein said tumour is selected from among the solid cancers, leukaemias and lymphomas.

6. Use according to claim 4 or 5, wherein said tumour is selected from among the T lymphoid leukaemias in adults (ALT) and the melanomas.

7. Use according to any one of claims 1 to 6, wherein said substance is arsenic trioxide.

8. Use according to any one of claims 1 to 6, wherein said substance is melarsoprol.

9. Use according to any one of claims 1 to 6, wherein said substance is zVAD.

10. Use according to any one of claims 1 to 9, wherein said agent which induces overexpression of the protein PML is an interferon such as interferon α, β or γ.

11. Use according to claim 10, wherein said agent inducing overexpression of the protein PML is interferon α.

12. Use according to claim 10, wherein said agent inducing overexpression of the protein PML is interferon β.

13. Use according to claim 10, wherein said agent inducing overexpression of the protein PML is interferon γ.

14. Use of at least one substance selected from the arsenic compounds, provided that the arsenic compounds are not arsenic sulphide compounds, compounds having the same biological properties as arsenic, and inhibitors and/or substrates of caspases, combined with an interferon, for the production of a medicament intended to induce the death of undesirable cells and/or stimulate an immune reaction, the administration of said substance and the administration of said interferon being simultaneous or sequential.

15. *In vitro* process for inducing the death of undesirable cells, comprising bringing said undesirable cells into contact with a substance selected from the arsenic compounds, compounds having the same biological properties as arsenic, and inhibitors and/or substrates of caspases, said substance being combined with the protein PML and/or an agent that induces overexpression of the protein PML, preferably an interferon.

16. Pharmaceutical composition containing
a) either at least one inhibitor and/or substrate of caspases, combined with:
- at least one arsenic compound or a compound having the same biological properties as arsenic;
- and/or the protein PML;
- and/or at least one agent that induces overexpression of the protein PML, such as an interferon;
in the presence of a pharmaceutically acceptable carrier;
b) or at least one arsenic compound, provided that the arsenic compound is not an arsenic sulphide compound, or a compound having the same biological properties as arsenic, combined with the protein PML and/or at least one agent that induces overexpression of the protein PML, such as an interferon, in the presence of a pharmaceutically acceptable carrier.

17. Kit comprising
a) a pharmaceutical composition (1) containing at least one inhibitor and/or substrate of caspases, combined with a pharmaceutically acceptable carrier;
- and/or a pharmaceutical composition (2) containing at least one arsenic compound, provided that the arsenic compound is not an arsenic sulphide compound, or a compound having the same properties as arsenic, combined with a pharmaceutically acceptable carrier; and
b) a pharmaceutical composition (3) containing the protein PML combined with a pharmaceutically acceptable carrier;
- and/or a pharmaceutical composition (4) containing at least one agent that induces overexpression of the protein PML, such as an interferon, combined with a pharmaceutically acceptable carrier;
said pharmaceutical compositions being intended for simultaneous or sequential administration.

18. Use of at least one substance, other than arsenic compounds, which promotes the targeting of the protein PML towards the nuclear bodies and/or the stabilisation thereof, for the production of a medicament intended to induce the death of undesirable cells and/or stimulate an immune reaction.

19. Use according to claim 18, wherein said substance is selected from among the inhibitors and/or substrates of caspases.

20. Use according to claim 15, wherein said substance is zVAD.
